# EUROPEAN PATENT SPECIFICATION

(11) **EP 2 139 453 B1**
(45) Date of publication and mention of the grant of the patent: **04.05.2011**
(21) Application number: 08718742.3
(22) Date of filing: 14.03.2008
(51) Int. Cl.: A61K 9/10, A61K 31/59, A61P 17/06

(54) **POLYAPHRON TOPICAL COMPOSITION WITH VITAMIN D**
TOPISCHE POLYAPHRON-ZUSAMMENSETZUNG MIT VITAMIN D
COMPOSITION TOPIQUE DE POLYAPHRON AVEC VITAMINE D

(30) Priority: 15.03.2007 EP 07251084
(43) Date of publication of application: 06.01.2010
(73) Proprietor: Drug Delivery Solutions Limited, Leatherhead, Surrey KT22 7RY (GB)
(72) Inventor: WHEELER, Derek, Alfred, Leatherhead Surrey KT22 7RY (GB); STEELE, David, Fraser, Leatherhead Surrey KT22 7RY (GB)
(74) Representative: Hayes, Adrian Chetwynd
(86) International application number: PCT/GB2008/000900
(87) International publication number: WO 2008/110819

(56) References cited:
- WO-A-97/32559
- WO-A-2005/011643
- POYNER T ET AL: "LONG-TERM TREATMENT OF CHRONIC PLAQUE PSORIASIS WITH CALCIPOTRIOL" JOURNAL OF DERMATOLOGICAL TREATMENT, BASINGSTOKE, GB, vol. 4, no. 4, 1993, pages 173-177, XP000905338 ISSN: 0954-6634
- DEREK WHEELER: "High internal phase dispersions" CONFERENCE: COSMETICS AND COLLOIDS, [Online] 15 February 2005 (2005-02-15), pages 1-12, XP002445510 Retrieved from the Internet: URL:www.soci.org/SCI/groups/col/2005/repor ts/pdf/gs3257_wheeler.pdf> [retrieved on 2007-08-02]
- DARREN M ASHCROFT ET AL: "SYSTEMATIC REVIEW OF COMPARATIVE EFFICACY AND TOLERABILITY PF CALCIPOTRIOL IN TREATING CHRONIC PLAQUE PSORIASIS" BRITISH MEDICAL JOURNAL, LONDON, GB, vol. 320, 2000, pages 963-967, XP009077880 ISSN: 0267-0623

## Description

The present invention relates to a topical composition comprising at least one vitamin D or vitamin D analogue.

It is known for compositions comprising vitamin D or vitamin D analogues to be used for the treatment of a number of skin conditions.

For example, EP-B-474,517 discloses the use of compositions containing one or more 1a-hydroxylated-19-norvitamin D compounds with a triple bond in the side chain in the treatment of psoriasis.

US 4,871,723 discloses a process for treating psoriasis by topically applying a composition comprising vitamin D and a wax carrier. Specifically, US 4,871,723 discloses a composition comprising a) a pharmaceutically effective amount of an active-type vitamin D₃, b) a solvent selected from fatty acid esters, higher alcohols with 10 or more carbons and propylene carbonate and c) an oily carrier selected from white vaseline, yellow vaseline and liquid paraffin.

US 2005/002546 A1 discloses a pharmaceutical composition comprising an active vitamin D compound in emulsion pre-concentrate formulations, as well as emulsions and sub-micron droplet emulsions produced therefrom. In particular, the pharmaceutical compositions of US 2005/002546 A1 comprise
(a) a lipophilic phase component;
(b) one or more surfactants; and
(c) an active vitamin D compound.

The surfactant or surfactants are suitably present in an amount of from 1% to 90% by weight based on the total weight of the composition, and preferably from about 5% to about 85% by weight based on the total weight of the composition.

Presently available compositions contain relatively high concentrations of vitamin D or vitamin D analogues and surfactants which often lead to skin irritation and worsening of psoriasis. For example, in 1996 the Food and Drug Administration of America required that the label included in Dovonex (calcipotriene)- a product containing 0.005% calcipotriol be amended to indicate that approximately 25% of patients experienced skin irritation, and approximately 10% worsening of psoriasis. In addition, it has been reported that some patients treated with Dovonex have developed hypercalcaemia (see, for example, Hardman KA, Heath DA, Nelson HM Hypercalcaemia associated with calcipotriol (Dovonex) treatment. BMJ. 1993 Apr 3;306(6882):896-896).

There is a need to formulate an improved composition suitable for topical application which addresses at least some of the problems of the prior art.

US-A-2006/0228408 discloses oral drug delivery systems comprising a biliquid foam which comprises a poorly water-soluble drug such as vitamin D. The compositions disclosed in US-A-2006/0228408 are suitable for oral delivery for which purpose the level of continuous phase (typically water) must be kept to a minimum.

The present inventors have now developed a new topical composition comprising at least one vitamin D or vitamin D analogue. The present inventors have surprisingly found that such compositions have an enhanced dermal diffusion rate and/or improved stability compared to known compositions. Such compositions also have an appropriate viscosity such that they are useful for topical application. The compositions also have an agreeable skin feel during and after topical application (which helps to ensure good patient compliance with the treatment regime).

Accordingly, the present invention provides a topical composition comprising a continuous phase and at least one discontinuous phase, said composition comprising at least one polyaphron dispersion and at least one vitamin D or vitamin D analogue.

According to another aspect of the present invention there is provided a composition as described herein for use in the treatment of psoriasis.

According to another aspect of the present invention there is provided a composition as described herein for use in the manufacture of a medicament for the treatment of psoriasis.

According to a further aspect the present invention provides a composition as described herein for use in a method of treatment of the human or animal body by therapy.

According to a further aspect there is provided a method of treatment or prophylaxis of psoriasis in a subject which comprises topically applying to.a subject an effective amount of a composition as herein described.

In the following description, the meaning of the terms used are as follows: by hydrophilic phase or solvent is meant a liquid phase comprising water, comprising water together with other water-miscible liquids, or comprising a non-aqueous liquid which is miscible with water. By hydrophobic phase or solvent is meant a phase comprising pharmaceutically acceptable liquids such as oils that are immiscible or substantially immiscible with the hydrophilic phase. By immiscible liquids is meant that when mixed together, they separate to form two distinctly separate liquid phases sharing a well-defined interface. By substantially immiscible is meant that two liquids mixed as above having a well-defined interface between two phases where each phase may nevertheless contain small quantities of dissolved molecules of the other phase.

According to another aspect of the present invention there is provided a method of making the composition as described herein comprising the following steps:
(i) providing a hydrophilic solvent, optionally comprising at least one vitamin D or vitamin D analogue and/or a surfactant;
(ii) providing a hydrophobic solvent optionally comprising at least one vitamin D or vitamin D analogue and/or a surfactant;
(iii)mixing the hydrophilic solvent with the hydrophobic solvent under suitable conditions to form the compositions comprising at least one polyaphron dispersion and at least one vitamin D or vitamin D analogue.

Advantageously, the compositions of the present invention have an enhanced dermal permeation of the active agent compared to known compositions.. Thus, lower levels of vitamin D and vitamin D analogue may be required in the compositions of the present invention in order to achieve beneficial treatment results. As a result of having lower levels of vitamin D in the compositions of the present invention the likelihood of causing skin irritation is reduced.

A further particular advantage is that the compositions have good long term stability even at elevated temperature (40°C). Preferably, the compositions may contain water. This may be useful for dissolving water-soluble additives such as water-soluble preservatives, antioxidants, water-soluble permeation enhancers and the like.

A further advantage is that compositions of the present invention are typically manufactured at room temperature without the need to apply heat, making it less likely that actives will be damaged in the composition.

Advantageously the composition of the present invention has low antrophogenic potential (i.e. preferably use of this compound when applied to the skin causes less skin thinning compared to prior art compositions).

A yet further advantage of the composition of the present invention is that it need not comprise a high level of surfactant. In high concentrations surfactants are known to cause skin irritation. It is therefore desirable to keep the surfactant level to a minimum when applied to skin, and particular to damaged skin such as in case of psoriasis. Preferably the compositions of the present invention comprise less than 4% by weight of surfactant, more preferably less than 3%, more preferably still less than 2% by weight of the total composition.

By polyaphron dispersion as used herein is meant a particular kind of hydrophilic liquid-in-hydrophobic liquid or hydrophobic liquid-in-hydrophilic liquid dispersion comprising (a) a hydrophilic liquid miscible phase, (b) a second hydrophobic phase being immiscible or substantially immiscible with the first phase and (c) one or more surfactants, wherein the dispersed phase is in the form of small (e.g. micron to sub-micron diameter, but more usually at least 1 micron diameter) droplets, and the whole having the following characteristics, which distinguish polyaphron dispersions from conventional or common emulsions or other types of dispersion:
1. They are capable of existing in a stable form wherein the volume fraction of the dispersed phase (φᵢₚ) is greater than 0.7 and can be as high as 0.97. (φᵢₚ is the volume ratio of discontinuous to continuous phase expressed as a fraction).
2. The microscopic appearance of polyaphron dispersions where φᵢₚ is greater than 0.7 is of an aggregate of individual droplets, pushed closely together into polyhedral shapes, resembling the appearance of a gas foam. In this form, the dispersion has gel-like properties and is referred to as a Gel Polyaphron Dispersion (GPD).
3. Stable polyaphron dispersions can be formed with a surfactant concentration less than 3% and more typically less than 2% by weight of the total composition.
4. Gel Polyaphron Dispersions (as described in 2 above) can be diluted to any extent by the addition of more continuous phase without the addition of more surfactant, when the gel-like properties disappear. Once φᵢₚ has been reduced to below 0.7, the individual droplets of internal phase become separated to take the form of spherical droplets, which remain stable and intact but which may nevertheless join together in - loose associations and float to the top or sink to the bottom of the diluted dispersion (depending on the relative densities of the two phases). In this diluted form each droplet is referred to as a Colloidal Liquid Aphron (CLA). Simple shaking of the diluted dispersion instantly causes a homogeneous, stable dispersion of Colloidal Liquid Aphrons to re-form.

Each of the above characteristics and a combination of them clearly differentiate the polyaphron dispersions of the present invention from conventional emulsions, and other dispersion types which do not have all of those characteristics. Polyaphron dispersions are disclosed in the following literature references by Sebba: "Biliquid Foams", J. Colloid and Interface Science, 40 (1972) 468-474 and "The Behaviour of Minute Oil Droplets Encapsulated in a Water Film", Colloid Polymer Sciences, 257 (1979) 392-396, Hicks "Investigating the Generation, Characterisation, and Structure of Biliquid Foams", PhD Thesis, University of Bristol, 2005, Crutchley "The Encapsulation of Oils and Oil Soluble Substances Within Polymer Films", PhD Thesis, The University of Leeds, 2006 and Lye and Stuckey, Colloid and Surfaces, 131 (1998) 119-136. Aphrons are also disclosed in US-A-4,486,333 and WO 97/32559.

Polyaphron dispersions are sometimes referred to as 'Biliquid Foams', 'High Internal Phase Emulsions (HIPEs)', 'High Internal Phase Ratio Emulsions (HIPREs)' and 'Gel Emulsions'. In US 5,573,757 a composition comprising a polyaphron dispersion is described as "a viscoelastic gel". All descriptions that refer to dispersions having the characteristics described above are polyaphron dispersions of the present invention.

By "topical application" is meant application to human or animal, preferably to the skin, including for example the face, scalp, feet, limbs or trunk.

The present invention will now be further described. In the following passages different aspects of the invention are defined in more detail. Each aspect so defined may be combined with any other aspect or aspects unless clearly indicated to the contrary. In particular any feature indicated as being preferred or advantageous may be combined with any other feature or features indicated as being preferred or advantageous.

As described above polyaphron dispersions comprise a continuous phase, a discontinuous phase and a surfactant. The discontinuous phase is preferably a substantially hydrophobic internal phase, commonly known as an oil internal phase. Preferably, the discontinuous phase comprises a pharmaceutically acceptable oil phase.

Examples of oils which may be used in the present invention include almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, shark liver oil, squalane, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, isopropyl myristate, isopropyl isostearate, isopropyl palmitate, modified triglycerides, caprylic/capric glycerides, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl trilinoleate, glyceryl trilinolenate, glyceryl trioleate, glyceryl triundecanoate, linoleic glycerides, saturated polyglycolized glycerides, synthetic medium chain triglyceride containing primarily C₈-C₁₂ fatty acid chains, medium chain triglycerides, long chain triglycerides, modified triglycerides, fractionated triglycerides, and mixtures thereof.

Suitably the discontinuous phase comprises monoglycerides, diglycerides or triglycerides.

It will be understood that other suitable oils may be used in the present invention.

In a preferred embodiment, the discontinuous phase comprises or is caprylic/capric triglyceride and/or isopropyl myristate (IPM).

The discontinuous phase may, for example, confer an emollient, occlusive, moisturising, conditioning or other cosmetic or pharmaceutical benefit to the skin. It may also increase the viscosity of the composition and may confer solvency to the active or actives, and may contain materials providing a heating or cooling effect when applied to the skin (for example capsaicin or menthol).

The composition may comprise at least 1 % by weight of the discontinuous phase, preferably 1 to 80% by weight, more preferably from 10 to 80% by weight and most preferably from 25% to 80% by weight, based on the weight of the total composition.

The continuous or external phase of the polyaphron dispersion is preferably formed of water, hydrophilic liquid or a mixture thereof. The continuous phase preferably comprises from 20 to 99 wt%, more preferably from 20 to 75% and most preferably from 50 to 75% water based on the weight of total composition. In one embodiment the continuous phase preferably comprises at least 25%, more preferably at least 50% by weight of water based on the weight of total composition. When any part of the continuous phase is aqueous, close control of the pH within suitable limits is required. Preferably, the pH will be in the range from 7.0 to 9.0, more preferably from 7.1 to 8.5, more preferably still from 7.2 to 8.0 These pH ranges have been found to enable the drug to be stable whilst still being suitable to put onto psoriaritic skin.

The compositions of the present invention may be non-aqueous, substantially non-aqueous or aqueous.

By the term "non-aqueous composition" as used herein is meant a composition which is effectively free of water and does not contain water that has been deliberately added. A "non-aqueous composition" as used herein preferably has less than 0.5% by weight water based on the total weight of the composition, more preferably less than 0.2% by weight water, most preferably less than 0.1% by weight of water based on the total weight of the composition.

By the term "substantially non-aqueous" as used herein is meant a composition comprising less than 5% by weight, more preferably less than 4.5% by weight, of water based on the total weight of the composition.

By the term "aqueous composition" is meant a composition comprising at least 5% by weight of water based on the total weight of the composition. Preferably, the aqueous composition comprises at least 10%, or at least 15% by weight of water based on the total weight of the composition. The aqueous composition may comprises at least 35%, or at least 40% by weight based on the total weight of the composition. For aqueous compositions, preferably the percentage of water is from 5% to 90% by weight, and more preferably from 5% to 50% by weight and most preferably from 8% to 20% by weight based on the total weight of the composition.

Preferably the composition of the present invention is dispersible in water. Preferably the composition of the present invention is dilutable in water. This increases the flexibility of use of the invention, for example in improving the application of the composition to the scalp through hair by leaving the hair wet, or from rinsing the preparation from any topical surface should the desire or need arise, or by the easy removal by rinsing of product from accidental contamination of clothing. These advantages improve the in-use experience of users and improve patient compliance.

The continuous phase may comprise or consist of water, a pharmaceutically acceptable liquid that is miscible or substantially miscible with water or a mixture thereof. The water miscible liquid is preferably a compound of formula R₁-OH where R₁ is C₁-C₁₀ alkyl and/or a compound of formula HO-R₂-H where R₂ is (C₂H₄)ₙ or (C₃H₆), where n is 1 to 100, preferably 1 to 25. R₁ and R₂ may be linear or branched. Preferably R₁ is C₁-C₄ alkyl, n is preferably 1 to 25. Preferably the continuous phase comprises water, propylene glycol, polypropylene glycol, polyethylene or polypropylene glycol, glycerol, ethanol, isopropanol or a mixture thereof. Where the continuous phase comprises polyethylene glycol, the polyethylene glycol is preferably a polyethylene glycol which is liquid at room temperature. The polyethylene glycol may, for example, contain from 1 to 12 ethylene or propylene oxide units and/or have a molecular weight of up to 600.

In one embodiment of the present invention the composition, and preferably the polyaphron dispersion, comprises from 0 to 60 wt%, preferably from 0 to 20 wt%, more preferably from 0 to 10 wt%, of a C₁ - C₄ alcohol, ethylene glycol, a liquid polyethylene glycol, propylene glycol, a liquid polypropylene glycol, diethylene glycol mono ethyl ether or mixtures thereof. One of the advantages of the composition of the present invention over prior art compositions is that high levels of alcohol are not needed as skin permeation enhancers. Alcohol is known to cause skin irritation when applied to damaged skin. The compositions of the present invention have the advantage that enhanced permeation can be achieved even without the need for alcohol to be present, or without the need for high levels of alcohol. The present inventors have surprisingly found that it is advantageous for the composition of the present invention to comprise from 0 to 25% by weight of alcohol, more preferably from 0 to 15% by weight based on the total weight of the composition. Preferably the alcohol is isopropanol. Such compositions have improved drug delivery properties.

It will be understood that other suitable hydrophilic solvents may be used in the continuous phase of the polyaphrons.

The composition may comprise at least 20 % by weight of the continuous phase, preferably 20 to 99% by weight, more preferably from 20 to 90% by weight and most preferably from 20% to 85% by weight, based on the weight of the total composition.

The surfactant used in the present invention may be incorporated into either or both phases of the polyaphron dispersion. Suitable surfactants include an alkyl polyglycol ether, an alkyl polyglycol ester, an ethoxylated alcohol, a polyoxyethylene sorbitan fatty acid ester, a polyoxyethylene fatty acid ester, an ionic or non-ionic surfactant, a hydrogenated castor oil/polyoxyethylene glycol adduct containing from 25 to 60 ethoxy groups a castor oil/polyoxyethylene glycol adduct containing from 25 to 45 ethoxy groups, a sorbitan fatty acid ester (for example Span 20 or Span 80), a block copolymer of ethylene oxide and propylene oxide (for example Pluronic L121 or Pluronic F68), or a mixture thereof.

It will be understood that other suitable surfactants may be used.

Preferably the compositions of the present invention comprise less than 4% by weight of surfactant, more preferably less than 3%, more preferably still less than 2% by weight of the total composition.

In the composition of the present invention at least one vitamin D or vitamin D analogue is present predominantly in the discontinuous phase.

The vitamin D analogue employed in the composition of the present invention may, for example, be calcipotriol, calcipotriol monohydrate, seocalcitol, calcitriol, calcipotriol hydrate, tacalcitol, maxacalcitol, paricalcitol, falecalcitriol, becocalcidiol, 1α,24S-dihydroxy-vitamin D2, 1(S),3(R)-dihydroxy-20(R)-[((-(2-hydroxy-2-propyl)-phenyl)-methoxy)-methyl]-9,10-seco-pregna-5(Z),7(E),10(19)-triene, or a mixture.thereof. More preferably, the vitamin D anolgue is calcipotriol, calcitriol, tacalcitol, maxacalcitol, 1a,24S-dihydroxy-vitamin D2, 1(S),3(R)-dihydroxy-20(R)-[((3-(2-hydroxy-2-propyl)-phenyl)-methoxy)-methyl]-9,10-seco-pregna-5(Z),7(E),10(19)-triene, or a mixture thereof. Most preferably, the vitamin D analogues are calcipotriol and calcipotriol hydrate. Other examples of suitable vitamin D analogues are described in US-B-6,753,013.

In one embodiment of the present invention, the vitamin D analogue is calcipotriol and/or calcipotriol monohydrate

Synthetic vitamin D analogues are preferred in the compositions of the present invention over naturally occurring vitamin D or vitamin D derivatives, since the therapeutic effects of the latter may be less selective for the treatment of skin diseases, such as psoriasis.

The composition of the present invention may comprise from 0.0001 to 0.05% by weight of vitamin D or vitamin D analogue, preferably from 0.001 to 0.01% by weight and more preferably from 0.0025 to 0.005% by weight of the total composition.

Preferably, the composition of the present invention may further comprise a gelling agent and/or a viscosity modifying agent such as a rheology modifying agent

The gelling agent may, for example, be selected from alginate gums or their salts, guar gum, locust bean gum, xanthan gum, gum acacia, gelatin, hydroxymethyl-cellulose hydroxyethylcellulose, hydroxypropyl-cellulose, carboxymethylcellulose or its salts, bentonites, magnesium aluminium silicates, "Carbomers" (salts of cross-linked polymers of acrylic acid), or glyceryl polymethacrylates or their dispersions in glycols. It will be understood that other suitable gelling agents and/or a viscosity modifying agent or a rheology modifying agents could be used. Additionally, the inventors have discovered that some of the gelling agents (for example, carbomers) may also function as a chemical buffering agents thus preventing unwanted variation in the pH of the composition during storage and use.

Preferably, the composition of the present invention comprises from 0.05 to 5.0% by weight of a gelling agent, preferably from 0.1 to 2.0% by weight and more preferably from 0.2 to 1.0% by weight of the total composition.

In one embodiment of the present invention the composition has the consistency of a gel. The gel like consistency may be formed from one, two, three or more polyaphron dispersions. Preferably, the composition is an aqueous composition.

The composition of the present invention may be used in a method of treatment of the human or animal body by therapy. Further, the composition of the present invention may be used in the treatment of psoriasis. Also the composition of the present invention may be used in the manufacture of a medicament for treatment of psoriasis.

Preferably the composition is applied in unit dosage form.

In one embodiment of the present invention, the composition as described herein may be applied to the scalp or other skin surface through hair. Preferably in this embodiment the hair is wetted (for example by use of water with or without shampoo, and then towel dried). The product may then be applied to the scalp in a suitable amount and then massaged into the scalp through the hair. The hair may then be left to dry naturally or dried using a hair dryer. Advantageously, the water-dispersible form of the formulation enables an even distribution of the actives on the skin using this process. Alternatively, or additionally, the composition may be massaged into the scalp through dry hair and left for a suitable period (which may be 8 to 12 hours) after which the excess or reminder may be rinsed out with water with or without shampoo.

Preferably the composition is applied to an animal in unit dosage form.

The compositions of the present invention may also contain other additives such as one or more of a preservative (for instance to prevent microbiological spoilage), a buffering agent (for the control of pH and to avoid instability and damage to the skin's acid mantle), an antioxidant,a permeation enhancer, a sunscreening agent, a cooling agent, a warming agent, an antipruritic agent , an aesthetic agent, a cosmetic masking agent, a foaming agent, a fragrance, a colouring agent, or an emollient oil and including mixtures thereof.

These additives may be included in the continuous or the discontinuous phase of the polyaphron dispersion.

It will be understood that the inclusion of these additives will be at the levels and with the type of materials which are found to be effective and useful. Care needs to be taken in the choice and amount of these additives to prevent compromise to the other performance advantages of the present invention.

The composition of the present invention may further comprise additional active agents, for examples, steroids or corticosteroids. Desirably, the composition does not comprise a corticosteroid.

In one embodiment of the present invention, the composition comprises at least one polyaphron dispersion, at least one vitamin D or vitamin D analogue and optionally at least one corticosteroid.

In a particularly preferred composition the discontinuous phase is a caprylic/capric triglyceride, the continuous phase is demineralised water and the vitamin D analogue is calcipotriol. This composition may optionally comprise or not comprise a corticosteroid such as betamethasone dipropionate

According to one aspect of the present invention, there is provided a method of making the composition as described herein comprising the following steps:
(i) providing a hydrophilic solvent, optionally comprising at least one vitamin D or vitamin D analogue and/or a surfactant;
(ii) providing a hydrophobic solvent optionally comprising at least one vitamin D or vitamin D analogue and/or a surfactant;
(iii) mixing the hydrophilic solvent with the hydrophobic solvent under suitable conditions to form the composition comprising at least one polyaphron dispersion and at least one vitamin D or vitamin D analogue.

In one embodiment of the present invention, the vitamin D or vitamin D analogue is mixed into the hydrophobic solvent prior to the polyaphron formation step. Alternatively, or additionally, the vitamin D or vitamin D analogue may be mixed into the continuous phase prior to the polyaphron formation step.

Suitable methods for preparing polyaphron dispersions are described in US-A-4486333. It will be understood by those skilled in the art that other manufacturing methods may be used, as appropriate.

According to another aspect of the present invention, there is provided a method of making the composition as described herein comprising the following steps:
preparing a first polyaphron dispersion comprising a vitamin D or vitamin D analogue;
preparing a second polyaphron dispersion comprising a an active agent, such as one or more of a sunscreening agent, a cooling agent, a warming agent, an antipruritic agent , an aesthetic agent, a cosmetic masking agent, a foaming agent, a fragrance, a colouring agent, an antioxidant or an emollient oil and including mixtures thereof;
and mixing together said first and second polyaphron dispersions to form the composition.

The method may further comprise:
preparing a third or further polyaphron dispersion comprising an further active agent,
and mixing said third or further polyaphron with said first and second polyaphron dispersions to form the composition.

The composition as described herein may be delivered to the skin in the form of an aerosol or a spray. For example EP 1,575,542 teaches the incorporation of a biliquid foam into aerosols. The composition of the present invention may be incorporated into aerosols by the addition of water and propellant gas (for example butane).

The composition of the present invention may be in the form of, for example, a lotion or a cream. The composition may be stored in any suitable jar, tube, bottle, sachet, aerosol, spray applicator or pump action sealed container. Advantageously in order for the compositions to remain stable the containers preferably prevent oxygen from entering the container. Preferably they the compositions of the present invention are sealed in air tight containers in order to prevent degradation of the compositions prior to use.

The present invention will now be described further, by way of example only, with reference to the following figure, in which:

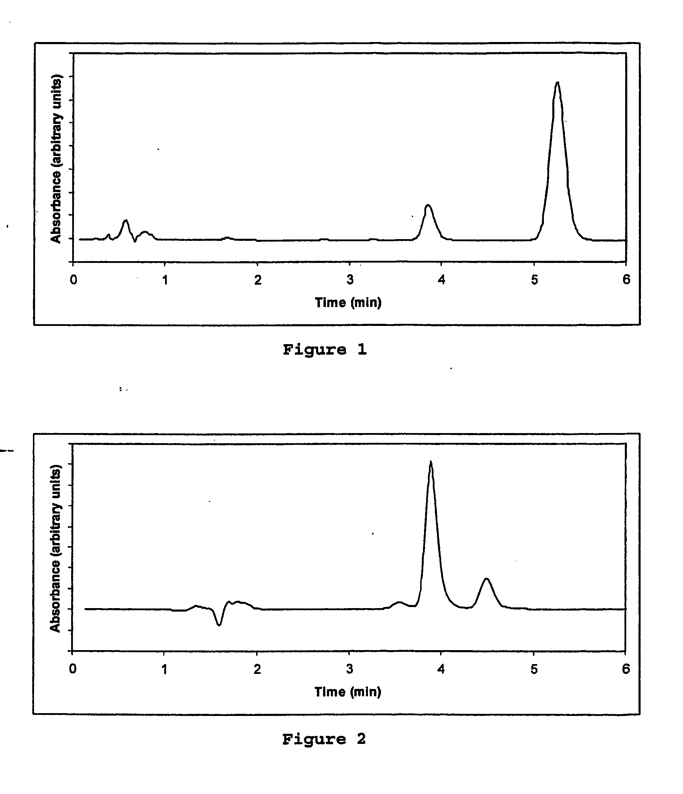

**Figure 1** is a HPLC chromatogram of a stable sample (Example 4). Peak for calcipotriol is at 3.9 min. No evidence of degradation products is observed. HPLC conditions are: Column:NovaPakC18, 4µ particle size, 3.9 x 150mm column (Waters), Mobile phase: 55% acetonitrile in water. Flow rate: 1ml/minute. Column Temperature: 25°C.
**Figure 2** is a HPLC chromatogram of the degradation of calcipotriol as evidenced by the presence of extraneous peaks (4.5 min and 3.6 min) either side of the main calcipotriol peak (3.9 min). No additional active agent is present here. HPLC conditions are: Column:NovaPakC18, 4µ particle size, 3.9 x 150mm column (Waters), Mobile phase: 55% acetonitrile in water. Flow rate: 1ml/minute. Column Temperature: 25°C.

### Definition of stability.

For the present invention, a product is considered to be storage stable if it meets with the following criteria.

The product is stored in closed, airtight glass containers with headspace comprising no more than 5% by volume of the total usable volume of the container.

The product and the container as defined above are stored at a constant temperature of 40°C in a standard laboratory oven (for example, Heraeus 'Function Line' air circulating oven model UT6, temperature control ± 0.3°C at 150°C).

The product is examined at the end of the examination period. The examination period is at least 3 months and preferably at least 6 months from the start date of storage.

The pass criteria are as follows:

| Procedure | Pass Criteria |
|---|---|
| Visual comparison of the appearance of the stored sample compared to a standard sample stored at 20°C for the same period. | Visual assessment indicates very little, if any, difference between stored sample and standard. In particular, appearance of the sample is uniform throughout with no sign of separation into two or more distinct phases. |
| A microscopic examination at a magnification of at least 200X comparing the microscopic appearance of the stored sample with a stored image of its appearance at the date of commencement of storage | Examination indicates very little, if any change in the size and size distribution of polyaphron droplets, with no sign of separated phases. |
| Analysis of the pharmacologically active components of the formulation by the extraction and HPLC method given hereinunder | Each active shall not have diminished by more than 5% by weight of the original content at the date of commencement of the storage test after 3 months of storage. Known decomposition products of the actives, if any such are present, collectively constitute no more than 5% of the original active based upon area under the curve measurements. See Figures 1 and 2 for further clarification. |

Any stored samples that meet the above criteria under the test conditions given above are considered to be storage stable for the purposes of this invention.

The FDA's 'Guidance for Industry Q1A (R2) Stability Testing of New Drug Substance and Products' although non-binding, specifies accelerated storage conditions that include storage at specific temperatures (e.g. 40°C) for a specific time (6 months) and at a controlled relative humidity (75% RH). The European Agency for the Evaluation of Medicinal Products, ICH Q1A (R2) 'Stability Testing Guidelines: Stability Testing of New Drug Substances and Products' specifies identical conditions for accelerated storage testing. The stability test method specified above for the purpose of this invention does not include provision for the control of relative humidity since storage takes place in closed glass containers whose walls and closures are impervious to the passage of water vapour.

The above definition of storage stable is definitive for the purposes of this invention.

The following Examples further illustrate the present invention.

### EXAMPLE 1

| ***Gel Polyaphron Oil Phase 1*** | **%w/w** |
|---|---|
| Solution comprising 0.00556% w/w | 90.0 |
| calcipotriol, 1% Tween 80 in caprylic/capric | |
| triglyceride (Miglyol 812 - Condea) | |
| | |

| ***Gel Polyaphron Aqueous Phase 1*** | |
|---|---|
| Poloxamer 188 (Pluronic F68 - BASF) | 1.0 |
| Demineralised Water | 9.0 |
| 50mM phosphate buffer | To pH 8.0 |

### Method of Manufacture of the Gel Polyaphron dispersion 1

A low form, 250ml laboratory beaker (internal diameter 6.5cm) was charged with sufficient aqueous (continuous) phase to make 30 g of gel polyaphron. This was stirred at 200 rpm with a four-bladed impeller having a diameter of 6.0 cm whilst adding the oil (discontinuous) phase dropwise from a Pasteur pipette. The rate of addition at the start of the process was slow (approximately one drop every 7 seconds) but was speeded up once 20% of the oil phase had been added so that the total time to make the gel polyaphron was approximately 20 minutes.

| ***Example 1*** | **%w/w** |
|---|---|
| Polyacrylic Acid Polymer (Ultrez 10 - Noveon) | 0.56 |
| Triethanolamine | To pH 7.5 |
| Demineralised Water | 55.04 |
| Gel Polyaphron 1 | 44.4 |

### Method of Manufacture of Example 1

The aqueous phase of the composition was made by pre-dispersing the Ultrez 10 into the water and then adding the triethanolamine until the pH had reached 7.7, by which time a clear gel had formed. The Gel Polyaphron dispersion 1 was then stirred into the gelled aqueous phase until homogeneously mixed.

Prior to the manufacture of each Gel Polyaphron dispersion, any active was dissolved in the appropriate phase by gentle stirring overnight with a magnetic stirrer at room temperature in a covered beaker.

### Stability Measurements

Stability measurements were made using the method outlined below.

The calcipotriol was extracted from the composition of Example 1 into isopropanol and assayed by HPLC under the conditions given below.

### HPLC conditions:

Column:NovaPakC18, 4µ particle size, 3.9 x 150mm column (Waters)
Mobile phase: 47% acetronitrile in water.
Flow rate: 1ml/minute.
Column Temperature: 25°C.
Calcipotriol Retention Time: 6.9 minutes

The results show that after 3 months storage at 40°C, the level of calcipotriol was 99% ±1% of the original level.

### EXAMPLE 2

| ***Gel Polyaphron Oil Phase 2*** | **%w/w** |
|---|---|
| Solution comprising 0.00556% w/w . calcipotriol, 1% laureth-4 in isopropyl myristate (IPM-NF - Inolex) | 90.0 |
| | |

| ***Gel Polyaphron Aqueous Phase 2*** | |
|---|---|
| Poloxamer 188 (Pluronic F68 - BASF) | 1.0 |
| Demineralised Water | 9.0 |
| 50mM phosphate buffer | To pH 8.0 |

### Method of Manufacture of the Gel Polyaphron dispersion 2

A low form, 250ml laboratory beaker (internal diameter 6.5cm) was charged with sufficient aqueous (continuous) phase to make 30 g of gel polyaphron. This was stirred at 200 rpm with a four-bladed impeller having a diameter of 6.0 cm whilst adding the oil (discontinuous) phase dropwise from a Pasteur pipette. The rate of addition at the start of the process was slow (approximately one drop every 7 seconds) but was speeded up once 20% of the oil phase had been added so that the total time to make the gel polyaphron was approximately 20 minutes.

| ***Example 2*** | **%w/w** |
|---|---|
| Polyacrylic Acid Polymer (Ultrez 10 - Noveon) | 0.56 |
| Triethanolamine | To pH 7.5 |
| Demineralised Water | 55.04 |
| Gel Polyaphron 2 | 44.4 |

### Method of Manufacture of Example 2

The aqueous phase of the composition was made by pre-dispersing the Ultrez 10 into the water and then adding the triethanolamine until the pH had reached 7.7, by which time a clear gel had formed. The Gel Polyaphron dispersion 2 was then stirred into the gelled aqueous phase until homogeneously mixed.

Prior to the manufacture of each Gel Polyaphron dispersion, any active was dissolved in the appropriate phase by gentle stirring overnight with a magnetic stirrer at room temperature in a covered beaker.

### EXAMPLE 3

The composition of Example 1 was tested for steady state of flux rate (measured over 12 hours) through silicone membranes in Franz diffusion cells for the calcipotriol. This is a recognised in vitro test correlating to permeation through the skin. For comparison the commercial products Dovonex and Dovobet were also tested.

| **Composition** | **Calcipotriol Flux Rate ng cm⁻² hr⁻¹** | **Ratio of Rate to that of Dovonex** |
|---|---|---|
| Example 1 Composition | 111.2 | 1.33 |
| Dovonex | 83.3 | 1.00 |
| Dovobet | 74.5 | 0.89 |

### EXAMPLE 4

| ***Gel POLYAPHRON DISPERSION 1*** | |
|---|---|
| ***Oil Phase*** | **%** |
| Calcipotriol solution* (0.02125%) in isopropyl | 89.10 |
| myristate (Lexol IPM NF - Inolex) | |
| Laureth-4 (Volpo L4 - Croda) | 0.90 |
| | |

| ***Aqueous Phase*** | |
|---|---|
| Poloxamer 188 (Pluronic F68 - BASF) | 1.00 |
| Demineralised Water | 9.00 |
| | |

| ***Gel POLYAPHRON DISPERSION 2*** | |
|---|---|
| ***Oil Phase*** | **%** |
| Isopropyl myristate (Lexol IPM NF - Inolex) | 89.10 |
| Laureth-4 (Volpo L4 - Croda) | 0.90 |
| | |

| ***Aqueous Phase*** | |
|---|---|
| Poloxamer 188 (Pluronic F68 - BASF) | 1.00 |
| Demineralised Water | 9.00 |

| ***Gel POLYAPHRON DISPERSION 3*** | |
|---|---|
| ***Oil Phase*** | **% w/w** |
| Squalane (Olive-derived - A & E Connock) | 20.00 |
| Dimethicone (Q7-9120, 20 CP - Dow Corning) | 35.00 |
| Elastomer 10 (Dow Corning) | 5.00 |
| Cyclomethicone (STb5NP - Dow Corning) | 29.00 |
| Laureth-4 (Volpo L4 - Croda) | 1.00 |
| | |

| ***Aqueous Phase*** | |
|---|---|
| Poloxamer 188 (Pluronic F68 - BASF) | 1.00 |
| Demineralised Water | 9.00 |
| | |

| ***AQUEOUS GEL*** | |
|---|---|
| | **% w/w** |
| Polyacrylic acid (Ultrez 10 - Noveon) | 1.00 |
| Triethanolamine (TEA) | to pH 7.22 |
| Demineralised Water | qs to 100% |
| | |

| ***FINAL PRODUCT*** | **% w/w** |
|---|---|
| *GEL POLYAPHRON DISPERSION* 1 | 27.04 |
| *GEL POLYAPHRON DISPERSION* 2 | 22.96 |
| *GEL POLYAPHRON DISPERSION* 3 | 30.32 |
| Aqueous gel | 19.68 |

| | |
|---|---|
| ** final calcipotriol level 51.2* µ*g*/*g* | |

After storage for 3 months at 40°C the level of calcipotriol was 103% of the initial value. Storage stability testing is ongoing.

### Manufacturing Method

The method used was as described for Example 1 for both the polyaphron dispersion and the aqueous gel above except that the neutralised aqueous gel were added to the final mixture of the three gel polyaphron dispersions, 1, 2 and 3 and then mixed in by simple mixing until the product was a homogeneous mixture of the polyaphron dispersions.

By neutralised gel is meant the addition of triethanolamine (a base) to a dispersion of the polyacrylic acid to form a clear gel having a pH value of 7.5 ±0.2. The process of neutralisation of polyacrylic acid gels is well known to those skilled in the art.

### EXAMPLE 5

| ***Gel POLYAPHRON DISPERSION 1*** | |
|---|---|
| ***Oil Phase*** | **%** |
| Calcipotriol solution* (0.06440%) in a 3:2 w/w blend of isopropyl myristate (Lexol IPM NF - Inolex): Caprylic/Capric Triglyceride (Mygliol 812 - Condea) | 17.48 |
| Squalane (Olive-derived - A & E Connock) | 7.54 |
| DC 200 fluid (Dow Corning) | 19.96 |
| Dimethicone (Q7-9120, 100 CP - Dow Corning) | 15.02 |
| Cyclomethicone (STb5NF - Dow Corning) | 29.02 |
| Laureth-4 (Volpo L4 - Croda) | 0.99 |
| | |

| ***Aqueous Phase*** | |
|---|---|
| Poloxamer 188 (Pluronic F68 - BASF) | 1.00 |
| Demineralised Water | 8.99 |

| ***AQUEOUS GEL*** | |
|---|---|
| | **% w/w** |
| Polyacrylic acid (Ultrez 10 - Noveon) | 1.00 |
| Triethanolamine (TEA) | to pH 8.00 |
| Demineralised Water | qs to 100% |

| ***FINAL PRODUCT*** | **% w/w** |
|---|---|
| *GEL POLYAPHRON DISPERSION 1* | 40.07 |
| Aqueous gel | 59.93 |

| | |
|---|---|
| * *final calcipotriol level 45.1* µ*g*/*g* | |

After storage for 13 months at room temperature the level of calcipotriol was 105% of the initial value. Storage stability testing is ongoing.

### Manufacturing Method

The method used was as described for Example 1 for both the polyaphron dispersion and the aqueous gel above except that the neutralised aqueous gel were added to the final mixture of the three gel polyaphron dispersions, 1, 2 and 3 and then mixed in by simple mixing until the product was a homogeneous mixture of the polyaphron dispersions.

By neutralised gel is meant the addition of triethanolamine (a base) to a dispersion of the polyacrylic acid to form a clear gel having a pH value of 7.5 ±0.2. The process of neutralisation of polyacrylic acid gels is well known to those skilled in the art.

## Claims

1. A topical composition comprising a continuous phase, and at least one discontinuous phase, said composition comprising at least one polyaphron dispersion and at least one vitamin D or vitamin D analogue, wherein the vitamin D or vitamin D analogue is predominantly in the discontinuous phase.

2. A composition according to claim 1 wherein the vitamin D or vitamin D analogue is vitamin D, calcipotriol, seocalcitol, calcitriol, tacalcitol, maxacalcitol, paricalcitol, falecalcitriol, becocalcidiol, 1α, 24S-dihydroxy-vitamin D2, 1(S),3(R)-dihydroxy-20(R)-[((3-(2-hydroxy-2-propyl)-phenyl)-methoxy)-methyl]-9,10-seco-pregna-5(Z), 7(E), 10(19)-triene, or a mixture thereof.

3. A composition according to any one of the preceding claims which comprises the vitamin D or vitamin D analogue in an amount of from 0.0001 to 0.05 wt% of the total composition.

4. A composition according to any one of the preceding claims wherein the discontinuous phase comprises an oil.

5. A composition according to claim 4 wherein the oil comprises a monoglyceride, diglyceride or triglyceride or a mixture thereof.

6. A composition according to any one of the preceding claims comprising at least 20% by weight of continuous phase based on the total weight of the composition.

7. A composition according to any one of the preceding claims wherein the continuous phase comprises from 20 to 99% by weight of water based on the total weight of the composition.

8. A composition according to any one of the preceding claims wherein the continuous phase comprises water, a compound of formula R₁-OH where R₁ is C₁-C₁₀ alkyl and/or a compound of formula HO-R₂-H where R₂ is (C₂H₄) or (C₃H₆)ₙ where n is 1 to 100 or a mixture thereof.

9. A composition according to any one of the preceding claims further comprising a gelling agent.

10. A composition according to any one of the preceding claims further comprising a permeation enhancer.

11. A composition according to any one of the preceding claims which does not comprise a corticosteroid.

12. A composition as defined in any one of claims 1 to 11 for use in a method of treatment of the human or animal body by therapy, in particular by topical application.

13. A composition as defined in any one of claims 1 to 11 for use in the topical treatment of psoriasis.

14. Use of a composition as defined in any one of claims 1 to 11 in the manufacture of a medicament for the topical treatment of psoriasis.

15. A method of making the composition as defined in any one of claims 1 to 11 comprising the following steps:
(i) providing a hydrophilic solvent, optionally comprising at least one vitamin D or a vitamin D analogue, and/or a surfactant;
(ii) providing a hydrophobic solvent optionally comprising at least one vitamin D or a vitamin D analogue, and/or a surfactant;
(iii)mixing the hydrophilic solvent with the hydrophobic solvent under suitable conditions to form the composition comprising at least one polyaphron dispersion and at least one vitamin D or vitamin D analogue.

16. A composition as defined in any one of claims 1 to 11 which is stable, wherein stability is measured as a no more than 5% reduction in the amount of vitamin D or vitamin D analogue with respect to the original amount after 3 months of storage in a sealed glass container at 40°C.

## Patentansprüche

1. Topische Zusammensetzung umfassend eine kontinuierliche Phase und mindestens eine diskontinuierliche Phase, wobei die Zusammensetzung mindestens eine Polyaphrondispersion und mindestens ein Vitamin D oder Vitamin D-Analogon umfasst, wobei das Vitamin D oder Vitamin D-Analogon überwiegend in der diskontinuierliche Phase ist.

2. Zusammensetzung nach Anspruch 1, wobei das Vitamin D oder Vitamin D-Analogon Vitamin D, Calcipotriol, Seocalcitol, Calcitriol, Tacalcitol, Maxacalcitol, Paricalcitol, Falecalcitriol, Becocalcidiol, 1α,24S-Dihydroxy-vitamin D2, 1(S),3(R)-Dihydroxy-20(R)-[((3-(2-hydroxy-2-propyl)-phenyl)-methoxy)-methyl]-9,10-seco-pregna-5(Z),7(E),10(19)-trien oder eine Mischung davon ist.

3. Zusammensetzung nach einem der vorhergehenden Ansprüche, welche das Vitamin D oder Vitamin D-Analogon in einer Menge von 0,0001 bis 0,05 Gew.-% der Gesamtzusammensetzung umfasst.

4. Zusammensetzung nach einem der vorhergehenden Ansprüche, wobei die diskontinuierliche Phase ein Öl umfasst.

5. Zusammensetzung nach Anspruch 4, wobei das Öl ein Monoglycerid, Diglycerid oder Triglycerid oder eine Mischung davon umfasst.

6. Zusammensetzung nach einem der vorhergehenden Ansprüche, die mindestens 20 Gewichts-% der kontinuierliche Phase bezogen auf das Gesamtgewicht der Zusammensetzung umfasst.

7. Zusammensetzung nach einem der vorhergehenden Ansprüche, wobei die kontinuierliche Phase von 20 bis 99 Gewichts-% Wasser bezogen auf das Gesamtgewicht der Zusammensetzung umfasst.

8. Zusammensetzung nach einem der vorhergehenden Ansprüche, wobei die kontinuierliche Phase Wasser, eine Verbindung der Formel R₁-OH, wobei R₁ C₁-C₁₀-Alkyl ist, und/oder eine Verbindung der Formel HO-R₂-H, wobei R₂ (C₂H₄)ₙ oder (C₃H₆)ₙ ist, wobei n 1 bis 100 ist, oder eine Mischung davon umfasst.

9. Zusammensetzung nach einem der vorhergehenden Ansprüche weiter umfassend ein Geliermittel.

10. Zusammensetzung nach einem der vorhergehenden Ansprüche weiter umfassend einen Permeationsverstärker.

11. Zusammensetzung nach einem der vorhergehenden Ansprüche, welche kein Kortikosteroid umfasst.

12. Zusammensetzung wie in einem der Ansprüche 1 bis 11 definiert zur Verwendung in einem Verfahren zur Behandlung des menschlichen oder tierischen Körpers durch Therapie, insbesondere durch topische Anwendung.

13. Zusammensetzung wie in einem der Ansprüche 1 bis 11 definiert zur Verwendung in der topischen Behandlung von Psoriasis.

14. Verwendung einer Zusammensetzung wie in einem der Ansprüche 1 bis 11 definiert für die Herstellung eines Medikaments für die topische Behandlung von Psoriasis.

15. Verfahren zum Herstellen der Zusammensetzung wie in einem der Ansprüche 1 bis 11 definiert umfassend die folgenden Schritte:
(i) Bereitstellen eines hydrophilen Lösungsmittels, das gegebenenfalls mindestens ein Vitamin D oder ein Vitamin D-Analogon und/oder ein Tensid umfasst;
(ii) Bereitstellen eines hydrophoben Lösungsmittels, das gegebenenfalls mindestens ein Vitamin D oder ein Vitamin D-Analogon und/oder ein Tensid umfasst;
(iii) Mischen des hydrophilen Lösungsmittels mit dem hydrophoben Lösungsmittel unter Bedingungen, die geeignet sind, um die Zusammensetzung umfassend mindestens eine Polyaphrondispersion und mindestens ein Vitamin D oder Vitamin D-Analgon zu bilden.

16. Zusammensetzung wie in einem der Ansprüche 1 bis 11 definiert, welche stabil ist, wobei die Stabilität als eine Verringerung von nicht mehr als 5% der Menge an Vitamin D oder Vitamin D-Analogon in Bezug auf die ursprüngliche Menge nach 3 Monaten Lagerung in einem abgedichteten Glasbehälter bei 40 °C gemessen wird.

## Revendications

1. Composition topique comprenant une phase continue, et au moins une phase discontinue, ladite composition comprenant au moins une dispersion de polyaphrons et au moins une vitamine D ou un analogue de vitamine D, dans laquelle la vitamine D ou l'analogue de vitamine D est présent de manière prédominante dans la phase discontinue.

2. Composition suivant la revendication 1, dans laquelle la vitamine D ou l'analogue de vitamine D est la vitamine D, le calcipotriol, le séocalcitol, le calcitriol, le tacalcitol, le maxacalcitol, le paricalcitol, le falécalcitriol, le bécocalcidiol, la 1α,24S-dihydroxy-vitamine D2, le 1(S), 3(R)-dihydroxy-20(R)-[((3-(2-hydroxy-2-propyl)-phényl)-méthoxy)-méthyl]-9,10-séco-prégna-5(Z),7(E),10(19)-triène ou un de leurs mélanges.

3. Composition suivant l'une quelconque des revendications précédentes, qui comprend la vitamine D ou l'analogue de vitamine D en une quantité de 0,0001 à 0,05 % en poids de la composition totale.

4. Composition suivant l'une quelconque des revendications précédentes, dans laquelle la phase discontinue comprend une huile.

5. Composition suivant la revendication 4, dans laquelle l'huile comprend un monoglycéride, un diglycéride, ou un triglycéride ou un de leurs mélanges.

6. Composition suivant l'une quelconque des revendications précédentes, comprenant au moins 20 % en poids de phase continue sur la base du poids total de la composition.

7. Composition suivant l'une quelconque des revendications précédentes, dans laquelle la phase continue comprend 20 à 99 % en poids d'eau sur la base du poids total de la composition.

8. Composition suivant l'une quelconque des revendications précédentes, dans laquelle la phase continue comprend de l'eau, un composé de formule R₁-OH, dans laquelle R₁ représente un groupe alkyle en C₁ à C₁₀ et/ou un composé de formule HO-R₂-H dans laquelle R₂ représente un groupe (C₂H₄)ₙ ou (C₃H₆)ₙ, dans lequel n a une valeur de 1 à 100 ou un de leurs mélanges.

9. Composition suivant l'une quelconque des revendications précédentes, comprenant en outre un agent gélifiant.

10. Composition suivant l'une quelconque des revendications précédentes, comprenant en outre un agent augmentant la perméation.

11. Composition suivant l'une quelconque des revendications précédentes, qui ne comprend pas de corticostéroïdes.

12. Composition suivant l'une quelconque des revendications 1 à 11, destinée à être utilisée dans une méthode de traitement de l'organisme d'un être humain ou d'un animal par thérapie, en particulier par application topique.

13. Composition suivant l'une quelconque des revendications 1 à 11, destinée à être utilisée dans le traitement topique du psoriasis.

14. Utilisation d'une composition telle que définie dans l'une quelconque des revendications 1 à 11 dans la production d'un médicament destiné au traitement topique du psoriasis.

15. Procédé pour la préparation de la composition telle que définie dans l'une quelconque des revendications 1 à 11, comprenant les étapes consistant à :
(i) fournir un solvant hydrophile comprenant éventuellement au moins une vitamine D ou un analogue de vitamine D, et/ou un agent tensioactif ;
(ii) fournir un solvant hydrophobe comprenant éventuellement au moins une vitamine D ou un analogue de vitamine D, et/ou un agent tensioactif ;
(iii) mélanger le solvant hydrophile au solvant hydrophobe dans des conditions convenables pour former la composition comprenant au moins une dispersion de polyaphrons et au moins une vitamine D ou un analogue de vitamine D.

16. Utilisation d'une composition telle que définie dans l'une quelconque des revendications 1 à 11 qui est stable, dont la stabilité est mesurée par une réduction non supérieure à 5 % de la quantité de vitamine D ou d'analogue de vitamine D par rapport à la quantité initiale après 3 mois de stockage dans un récipient en verre clos hermétiquement à 40°C.
